# EUROPEAN PATENT SPECIFICATION

(11) **EP 1 913 871 B1**
(45) Date of publication and mention of the grant of the patent: **11.01.2017**
(21) Application number: 06425724.9
(22) Date of filing: 19.10.2006
(51) Int. Cl.: A61B 5/055, G06K 9/68, A61B 5/103, A61B 5/107, G01R 33/28, G06F 19/00

(54) **Apparatus for determining indications helping the diagnosis of orthopedical diseases**
Gerät zur Feststellung von Indikationen hilfreich zur Diagnose von orthopädischen Krankheiten
Appareil pour constater des indications pour aider le diagnostique des malaides orthopédique

(43) Date of publication of application: 23.04.2008
(73) Proprietor: Esaote S.p.A., 16152 Genova (IT)
(72) Inventor: Biglieri, Eugenio, 15024 Masio (AL) (IT); Satragno, Luigi, 16122 Genova (IT)
(74) Representative: Karaghiosoff, Giorgio Alessandro

(56) References cited:
- EP-A- 1 460 443
- EP-A- 1 640 737
- WO-A-2005/076026
- US-A- 5 772 595

## Description

The present invention relates to a method and apparatus for determining indications helping the diagnosis of orthopedical diseases.

Orthopedical diseases are continuously increasing as regards the effect one the world population and they are one of the fields in which medical methods have most developped.

The international importance in improving healhty conditions of patients suffering from diseases within the muscle-skeletal field is proved by the announcement of the initiative "Bone and Joint Decade: 2000-2010" made by the World Health Organization.

Orthopedic diseases are one of the most common reason of invalidity and involve very high social and medical costs, being estimated at about 215 billion dollars a year in the United States. Particularly diseases affecting the backbone are widely spread: 90% of people during their life will suffer from the painful disease known as "backache". The backache is known to be the most important cause limiting working capacities of middle-aged people and to be one of the greatest cause for requiring medical examinations and generally health controls.

In studying orthopedic, joint diseases or the like and particularly the ones affecting the backbone the magnetic resonance is considered to be a "gold standard" method and examinations carried out on the backbone by MRI are 30% of all MRI examinations carried out in the world.

MRI apparata intended to acquire images from anatomical regions of orthopedic interest are known and particularly also the ones dedicated to analyse the backbone both in the supine and orthostatic position. The apparatus that in its shape is inspired to an "orthopedic tilting examination table" allows to rotate the magnet together with the patient examination table. The patient that is comfortably lay on the examination table is first analysed in its supine position, in the upright position or, in case, in intermediate positions, wherein the backbone bears the natural load and the possible disease can be better identified.

So called dedicated systems known at present allow a spreading of the resonance in studying diseases affecting the backbone or other orthopedic anatomical regions but are not of great help to low invasive surgical operations since the operation is carried out with the patient in his lay position by force, whereas prostheses that can be applied, such as for example artificial intervertebral disks or other ones, will be stressed only when the upright position is taken or under stressing conditions of the normal position of the patient during his everyday life or working and/or sporting activity and this can led to a not negligible wrong positioning risk.

Methods for the diagnosis by the aid of computer are known, so called CAD computer aided diagnosis, involving diagnostic images to be processed such to highlight shapes and objects in the images and to obtain information about the type of highlighted object.

In order to obtain qualitative and/or quantitative information from images about a predetermined object or about a particular anatomical region, available computers use quite complex algorithms that force the specialist dealing with the treatment of a particular disease to send files relevant to the image or image series to a specialized institute that will process them then providing desired data that will allow the specialist to go on in treating the disease.

This system will led to an increase of costs and time for treating the patient considerably postponing the starting of the therapy since it involves an exchange of information between the personnel assigned to acquire images, personnel assigned to process images and the doctor evaluating images and data obtained by the processing in order to arrange a right therapy. The patent application EP 1 640 737 A2 discloses evaluating orthopedic disease progression using magnetic resonance imaging.

The problem is to realize a diagnostic indagation instrument in the orthopedic field. In studying the type of disease suffered by the patient, it is important for the doctor to have a complete instrument allowing to verify and quantify the presence of orthopedic diseases.

A further aim of the present invention is to develop a method and an apparatus for highlighting changes to the backbone in various postures and under different loading conditions, particularly the supine position or under resting condition of structures and the one with load. As an alternative the method allows to highlight changes to muscle skeletal structures of orthopedic interest, such as cartilages, bones, ligaments or the like under resting condition, under conditions with load and/or at intermediate positions.

The invention achieves the above aims by providing an apparatus for determining indications helping the diagnosis of orthopedic diseases comprising a section detecting and acquiring signals from a body under examination or from a particular anatomical region that in addition it is characterized in that the detecting section is a unit detecting images by nuclear magnetic resonance and in said apparatus there is further integrated a section for processing acquired images as regards image data and/or resonance signals, which processing section defines, from image data and/or resonance signals, values of one or more different numerical parameters indicating the presence or absence of an orthopedic disease and/or a measure of the evolution condition of said orthopedic disease.

Therefore in the above apparatus there are integrated both functionalities about the mere acquisition, generation, storing and displaying of MRI images typical of current MRI apparati, and functionalities processing image data that are typical of image processing systems with CAD functionalities (Computer Aidid Diagnosis), such as the automatic or semi-automatic recognition of objects represented in images, the automatic or semi-automatic recognition of qualitative, quantitative and/or morphologic and/or dynamic and/or geometric characteristics of objects represented in MRI images and/or possible classifications or predictions of said objects represented by specific areas or volumes of an image regarding predetermined characteristics, as well as the extraction of values of physic or physiologic parameters, whose processing functionalities are carried out under the "on line" mode by the scan, i.e. immediately after having acquired image data. Processing means are composed of one or more software modules independent one with the other and they can be combined in any combinations and can be interfaced one with the other and are composed of a software classificating and processing MRI image data, a 3D displaying and modelling software, a software for the quantitative analysis of parameters specific of the application method and a software supporting the decision about carrying out different steps of the examination and the analysis of results. Preferably said softwares work on a standard platform (PC/Windows) and are loaded and are executed by a processing hardware integrated in a dedicated MRI tomograph having characteristics optimized for such aim.

According to a further advantageous characteristic, the system provides means for storing a database of diagnostic images and processing data with CAD means relevant to each patient.

Storing means can be composed of means integrated or resident in the system computer or also of movable and portable storage media in combination with readers of said media provided in the system.

Storing means can also contain other kinds of data obtained by different techniques for acquiring diagnostic images, such as for example radiologic or ultrasound techniques, or the like such that it is possible to make a comparison regarding the image data with other parameters directly deriving from acquired signals and regarding processing results of said image data with processing results of signals acquired by other methods.

In addition to diagnostic data that is images and results of image processings, the patient database can comprise further data, such as image acquisition settings, used processing means, patient conditions at each image acquisition defined on the basis of other physic or physiologic parameters.

As regards the present invention characteristics thereof are applied both to two-dimensional images i.e. acquired along one or more section planes and to three-dimensional images, i.e. relevant to a certain volume of the body under examination.

Therefore it is necessary to develop MRI apparatuses dedicated to such applications allowing the implementation of high resolution three-dimensional methods, available for known existing apparatuses.

Therefore the present invention provides a new dedicated apparatus wherein various elements, particularly magnetic elements, will allow to carry out "quick" examinations required for carrying out three-dimensional methods, keeping advantages provided by low field permanent magnets in dedicated and compact apparatuses.

In the apparatus the integration of means intended to provide CAD functionalities, i.e. data treating programs, occurs by loading and executing said programs by a computer of the personal computer type or the like. Considering the fact that said computer executes software for generating and displaying images and that typically MRI image acquiring tomographs comprises computers in the form of personal computers or computers with dedicated hardware able to execute another code, the integration of functionalities of CAD systems in the tomograph from the constructive point of view requires at most an upgrade of mass and buffer memories and/or of the computation skill, i.e. of the processor and of elements cooperating with it, as well as the development of the CAD processing software.

According to an advantageous embodiment data acquired by the tomograph and subjected to the analysis by processing means having CAD functionalities are not only images, i.e. image data, but also intermediate data that can be obtained during MRI scans, such as for example the global acquired resonance signal hereinafter defined as resonance signal and/or also NMR relaxation measurements or the like.

As regards objective criteria determining indications helping the diagnosis about the presence or absence of an orthopedic disease and/or the evolution degree of the disease, the present invention provides the apparatus to be provided with processing sub-sections extracting from images alternatively or in combination measures regarding geometric parameters, particularly parameters highlighting changes to the muscle skeletal structures of orthopedic interest, such as cartilages, ligaments, bony tissue or the like, and with reference to a specific application field of the backbone structure between the supine position i.e. in the resting condition and the one with load by segmenting main structures and measuring geometric parameters concerning their reciprocal geometric position in both conditions, or in other intermediate ones, in order to make the diagnosis of possible pathologic state more objective.

Particularly MRI scanning means are shaped and have such a dimension that allow the acquisition of MRI images from anatomical regions of any joints or muscle skeletal region and particularly of the backbone for the lumbosacral and cervical portion. Therefore the apparatus is small, inexpensive and easy to be installed, allowing to treat the most spread orthopedic diseases such as herniae, stenoses, inflammatory states causing the widespread "backache".

The invention allows to obtain morpho-fuactional indications about orthopedic diseases by segmenting main muscle skeletal structures of orthopedic interest and by measuring geometric parameters regarding their reciprocal geometric position in both positions, the horizontal and upright ones, or in intermediate positions, such that the diagnosis of possible pathologic states such as herniae, stenoses and inflammatory states causing the widespread "backache" is more objective.

The method object of the present invention allows also to verify changes to the backbone due to the physiological load in order to define how to carry out at best microsurgery operations such as for example the partial or total replacement of the intervertebral disc by artificial prostheses. The method allows also to verify the right positioning of the prosthesis in the position under load.

CAD system allows to obtain morpho-functional information from images or MRI image sequences such to carry out an evaluation of the state of the disease and of the necessary therapeutic treatment, allowing to optimize the prescription of drugs and of possible surgical and physical therapies. The reduction of intervention time allows to accelerate the recovery of the patient and to reduce social and medical costs of orthopedic diseases.

The present invention provides a device integrating means for processing images and data for studying the anatomical region of the backbone under load and without load with means for acquiring them such to combine the carrying out of the medical examination with a management of images that in the whole is inexpensive, with the possibility of quickly and completely finding the most precocious trouble stages and so of making the most correct medical-surgical therapies.

The integration in a single diagnostic information acquiring system of means for detecting and identifying orthopedic diseases allows to reduce costs of such operations, both as regards the necessary hardware and regarding specific personnel and tools for processing image data. The optimization of tools evaluating image data about orthopedic diseases allows also to optimize image data acquiring parameters with reference to the searched disease and to the type of anatomical region and of the examination that has been carried out.

A sinergic effect is obtained both as regards realization time and costs, but also as regards the completeness and accuracy of the indagation that has been carried out, since various indagations are carried out in a dedicated way within the same system and in time immediacy.

Particularly the apparatus is for small clinics or private surgeries for which the use of services of a third party for processing image data is too much heavy both regarding time and costs and for which it is not possible to use specialized personnel.

According to an advantageous characteristic the invention provides for the sub-section determining the pathologic state of the anatomical region of the backbone to comprise means determining geometric parameters regarding the reciprocal space position of the main structures of the backbone, particularly vertebrae and spinal canal, in the supine conditions or resting condition and under load, or in intermediate positions and means for comparing said parameters with a reference value for discriminating the presence/absence of a pathologic condition and/or for the comparison with a reference scale for determining a parameter indicating the evolution state of the pathologic condition, which reference value for discriminating the presence /absence of the pathologic condition and/or which reference scale for determining the evolution state of the pathologic condition are included in a database of known clinical cases.

According to an alternative embodiment that can be provided also in combination with the previous embodiment the sub-section determining the pathologic condition of a specific orthopedic region, particularly of main muscle skeletal structures of orthopedic interest such as for example structures of the backbone, comprises means for determining numerical values of said pathologic condition both regarding the presence/absence of it and the evolution condition of the disease by means of the comparative analysis of the contrast of MR images such as the detection of maps in T1 or T2 or analysis of images by means of suitable contrast media, obtained at different stages of the pathologic condition from images relevant to patients being part of the database of known clinical cases and/or from images relevant to previous indagations carried out on the same patient.

An alternative third embodiment that can be used also in combination with one or more of the previous ones provides the sub-section determining the pathologic state of orthopedic structures such as joints or other ones and particularly of structures of the backbone to comprise means for determining numerical values of said pathologic condition both regarding the presence/absence of it and the evolution condition of the disease by processing image data by means of classification and/or predictive algorithms, which have been trained (training and testing) by means of image data regarding orthopedic structures and particularly the backbone of known clinical cases included in a database of known clinical cases.

Still an embodiment that can be provided individually or in combination with one or more of the previous embodiments provides a sub-section comprising means for segmenting images for determining subsets of image data and/or pixels and/or voxels and for identifying real objects represented in the image by said subsets of image data and/or pixels and/or voxels, the subset of pixels or voxels or image data being defined representing the main muscle skeletal structures and, regarding the anatomical region of the backbone, vertebrae and disc structures and dimensions and/or shape and/or geometries of said structures being determined. Therefore dimensions, geometry and morphologic characteristics of the muscle skeletal structures, particularly of vertebrae and disc structures are determined on the basis of dimensions, geometries and morphologic characteristics determined from images and particularly from the subset of pixels, voxels or image data that has been identified representing the vertebrae and disc structures in images. It is to be noted that what has been mentioned above can be applied to other anatomical regions such as for example joints where there is bony tissue and cartilage.

The sub-system determining the dimensions, geometry and/or the morphology of structures of the backbone or of joints can further comprise means for generating a virtual image reconstructed on the basis of image data processed by segmentation means. These means are known as rendering means and the invention provides them to be possibly combined with morphing and/or smoothing means.

According to a further characteristic of the invention, the sub-section determining dimensions, geometry and/or the morphology of muscle skeletal elements of orthopedic anatomical regions such as joints or vertebrae and intervertebral discs in the anatomical region of the backbone, comprises means for comparing dimension, geometric and/or morphologic data of the structures of said regions particularly of the backbone with dimension, geometric and morphologic data of a plurality of know clinical cases that are kept in a database of clinical cases. Said comparison provides information about the differences and indentities of dimensions and/or geometry and/or morphology of the structures of anatomical regions and/or of the backbone with respect to dimensions and/or geometries and/or morphology of said structures of one or more known clinical cases, and therefore it provides an indication about the presence /absence of an orthopedic disease and/or an indication about the evolution condition of said orthopedic disease.

As regards the comparison reference numerical values are determined according to the invention from a database of known clinical cases. This database comprises images of the anatomical region of interest and dimension and/or geometric and/or morphologic data of structures of the region are determined from said images by means determining dimension and/or the geometry and/or the morphology of structures of the anatomical region working by processing image data by means of segmentation and/or rendering and/or morphing and/or smoothing. At least one or more reference values are determined for discriminating the presence/absence of a disease and/or a scale of numerical values for different evolution degrees of the disease, and are compared with dimension, geometric and morphologic values of structures for determining an indication about the presence/absence and/or the evolution degree of a disease for example highlighting possible restrictions of the spinal canal or the instability of vertebrae in the case of the anatomical region of the backbone.

The above method provides a support for the specialist in correctly making the diagnosis in the orthpedic field and by objectively finding measured parameters, it allows a more accurate definition of the disease state and so of the necessary theraphy.

The method object of the present invention allows to effectively monitoring the evolution of the orthopedic disease during the therapeutic treatment, allowing to optimize the prescription of drugs, reducing costs, limiting side effects, aiding the recovery of the best condition of the patient to the complete advantage of his quality of life and reducing social and medical costs.

The change of known morphologic diagnosis methods in morpho-functional analysing methods allows to highlight and define the pathologic condition in a more simple way by means of an objective measurement of various parameters.

The acquisition of morphologic data providing different complementary and/or overlapping information of the examined anatomical region, and the acquisition of functional data that are essential for finding structures of interest, allow the functional diagnostic evaluation of orthopedic lesions.

As regards means determining numerical parameters described above, the image or images are often subjected to a segmentation and/or rendering process. The invention advantageously provides means for verifying the quality of the segmentation and/or rendering process. This is a critical process and it has to provide accurate and reliable data. Therefore according to a further characteristic of the invention, the apparatus comprises means for verifying the reliability in identifying the subset of pixels or voxels or image data representing in the image or in the set of image data a real object and particularly in the case of the backbone anatomical region, vertebrae, disc structures and/or the spinal canal. These verifying means comprise a database of dimension and/or geometric and/or volume and/or morphologic configuration data, i.e. of typical shapes of the backbone structures under specific conditions of absence or presence of a disease and/or a specific evolution degree of the disease. Dimensions, geometry, and/or volume and/or the morphology of structures under examination determined by sub-sections of means processing images of a patient under examination are compared with said typical dimension, geometric and/or volume data and/or with typical morphologies for said structures that are in the form of average values and/or a range of average values of dimension, geometric and/or volume and/or morphologic differences. In order to determine an objective result of the comparison a first maximum difference threshold is set, over which dimension, geometric and/or volume and/or morphologic data provided by sub-sections are considered as unreliable and incompatible ones. In such case dimension, geometric, volume or shape differences with respect to standard values are too much great and the probability of being errors or malfunctions is high. Verifying means are provided with a sub-section signalling or requiring the repetition of the process for determining dimensions and/or geometry and/or volume and/or morphologic characteritics and/or of the acquisition of MRI image or images or said verifying means are provided with a sub-section automatically commanding the repetition of the process for determining dimensions and/or geometry and/or volume and/or morphologic characteristics and/or the acquisition of MRI image or images.

It is possible to provide the process verifying the segmentation and/or the rendering to be iteratively repeated for a predetermined number of times that can be set as the user desires and/or till differences in dimension and/or geometry and/or volume and/or morphology came back within the maximum difference threshold.

The integration of means for acquiring, generating and displaying MRI images with analysing means having CAD functionalities according to the present invention involves the optimization of all parameters aiming at the best final result can be also "guided" by CAD itself. Analysing means with CAD functionalities comprise means for analysing acquired images as regards predetermined quality parameters of said image data and/or resonance signals and means for automatically changing acquisition settings and/or acquisition parameters of image data that are controlled by said means analysing image data on the basis of quality parameters of image data and/or of resonance signals from which they are determined.

Therefore the invention provides the detecting section, particularly the unit detecting images by nuclear magnetic resonance to be connected to the section processing acquired images by means of a feed-back line.

The processing section controls the change of parameters setting the detecting section and/or the choice or the change of image acquisition sequences carried out by the detection section for acquiring images on the basis of a verification of the quality of image data and/or of resonance signals with reference to characteristics of image data and/or resonance signals important for carrying out the processing processes carried out by the processing section.

According to a further characteristic, the detection section by the feed-back line controls the processing section in treating image data and/or resonance signals by one or more different sub-sections and according to a predetermined order on the basis of the greatest quality that can be obtained from image data and/or signals and/or the length of the acquiring process, of the length of the processing process and of the reliability of results of the image processing expressed in the form of statistic reliability or error parameters and/or in the form of fitness.

The invention can provide also mathematical means such as for example fuzzy algorithms or genetic algorithms for determining new parameters or new settings acquiring signals and/or image data such as for example based on the processing by means of genetic algorithms or other similar algorithms, which algorithms can also be guided by statistic algorithms selecting or predicting new values of acquisition parameters or of acquisition sequences that have a greater probability in providing better image data from the point of view of processing means.

In all above cases, the automatic or semi-automatic definition of the processing result that is the auxiliary indication of the diagnosis occurs by the comparison with a reference database of data of a general record of cases and/or of the record of cases related to the specific patient.

For making said database, for example, data obtained by means for determining the pathologic condition and/or the presence of damages to orthopedic structures can be associated to numerical variables whose values are defined with reference to a predetermined scale of numerical values and so can be used as variables for generating records that can be evaluated by expert systems or predictive systems, such as for example systems working on the basis of predictive algorithms of the artificial neural network type or statistic classifiers such as bayesian classifiers, Bayes networks, Support Vector Machines or the like.

Said algorithms are trained by data of a database of known clinical cases and to which there are provided parameters indicating the presence/absence of a disease and/or the evolution degree of the disease as input data determined by sub-sections determining dimensions geometries and/or the morphology of structures in the anatomical region of the backbone and possibly further data obtained by different examinations and/or personal data or the medical history of the patient.

According to a further characteristic, by means of a database of diagnostic images relevant to a specific patient, it is possible to repeat specific acquisitions of images or image sequences and corresponding processings with processing means having CAD functionalities in different time moments or along a time interval coinciding or comprising at least a part of a physiologic movement made by structures of a specific anatomical region. Moreover it is possible to acquire images or the image sequence of orthopedic structures involved in a specific movement in different stages of said movement that is from a starting or resting position of structures to a final position or under load of structures through one or more intermediate positions. Particularly when the backbone is analysed it will be possible to know reciprocal positions of vertebral bodies and of discs both at a resting position coinciding with a supine position, and in a loaded position coinciding with a substantially upright position of the body, and at intermediate positions allowing different stressing degrees of structures involved in the movement.

The fact of having a database of known clinical cases and/or of diagnostic images and/or of processing data relevant to each patient, acquired in different time moments even at relatively long time intervals, such as days, months or years allows to determine the evolution, i.e. the follow-up, of a disease by the comparison of image data and/or of processing results in order for example to determine the kind of intervention to be carried out and/or to verify the efficacy of a therapy in progress.

The possibility of precociously interfere with the therapy in progress and/or to plan microsurgical operations, for example for partially or totally replacing discs with artificial prostheses and to verify their correct positioning both in the resting condition with the patient in a lay position and in stressing condition taking the upright position it is possible by the fact that the processing of image data and/or of resonance signals for determing values of one or more different numerical parameters indicating the presence or absence of a disease and/or a measure of the evolution condition of said disease is carried out immediately after the acquisition of the image or images by nuclear magnetic resonance.

Further characteristics of the method are described in subclaims of the method.

Further improvements of the invention are object of subclaims.

Characterstics of the present invention and advantages deriving therefrom will be more clear from the following description of some embodiments with reference to annexed drawings wherein:
Figure 1 is a block diagram of the principle structure of the system according to the present invention.
Figure 2 is a block diagram of an example of segmentation process, wherein the conventional image segmentation process is integrated with morphologic and/or dynamic functional data typical of real objects reproduced in images.
Figure 3 is a block diagram of image processing means that are specifically provided in the system according to the present invention for the diagnostic help by computer in the rheumatologic field.
Figures 4, 5, 6 are a cross section according to a vertical plane median and parallel to surfaces of poles of the magnetic structure according to the present invention and in each one of such figures 3 to 7, the patient is shown in different positions obtained by one or more positioning and/or leaning and/or retaining and/or supporting means helping the positioning of the patient.

With reference to figures a diagnostic helping system in the orthopedic field by means of nuclear magnetic resonance image acquisition comprises an MRI apparatus for acquiring images, particularly diagnostic images, by nuclear magnetic resonance wherein a unit for processing acquired images is integrated providing to extract from images information about the state or conditions of objects reproduced in said images helping the diagnosis.

Particularly the system according to the present invention is intended for recognizing the presence/absence of orthopedic diseases and their evolution degree in the specific case of the backbone region. However above principles that will be described in more details can be applied to any anatomical regions subjected to orthopedic diseases

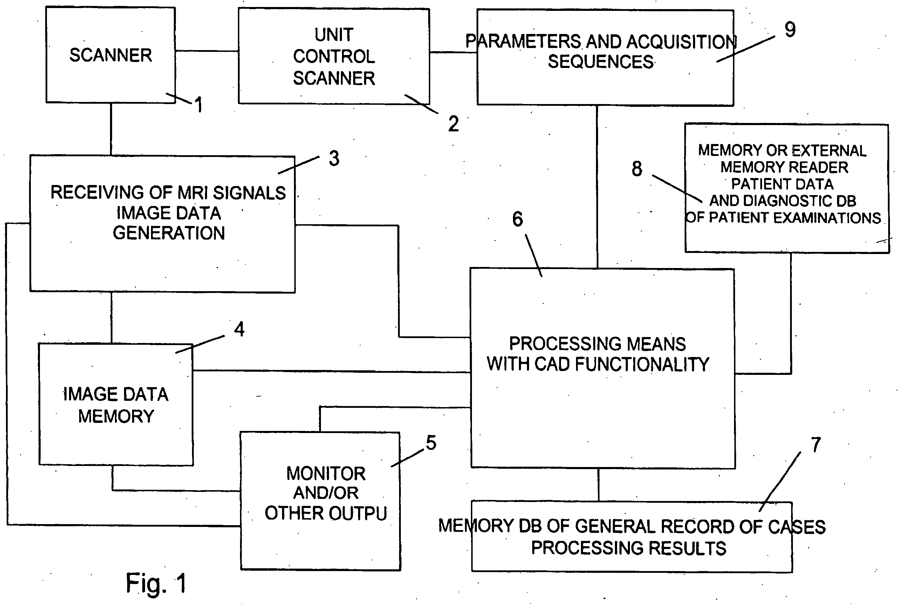

Figure 1 is a block diagram of a system according to the present invention. The system comprises a unit for acquiring images by nuclear magnetic resonance and a unit processing images with CAD functionality both grouped together in a single apparatus.

The scanner 1 represents the magnetic structure, gradient coils, receiving coil and the transmitting one and further possible devices or means for acquiring resonance signals. 2 and 3 indicate means controlling the scanner and means receiving signals and generating image data and both are generally composed of electronic devices. These means 2 and 3 can be also composed of software means loaded in a general hardware executing the software, such as a computer or also a personal computer.

Image data generated by the unit 3 are stored in a memory 4 and therefore they can be called up for being displayed at any moments by means of the monitor 5 or other output means or they can be immediately displaying apart from the storage.

The unit processing image data having CAD functionalities comprises processing means with CAD functionalities that are indicated by 6 and to which there are provided or which call up image data of one or more images to be subjected to processing processes from memory 4, or from the unit receiving and generating image data 3 and/or possibly even from the monitor or from further possible output means 5 when they allow it.

Processing results can be displayed on the monitor 5 or can be printed or personnel can access to them by other output means. Moreover in combination or as an alternative results are stored in a memory 7 of general record of clinical cases that has both the task of having data of various examinations for each patient available in order to allow the identification of the time evolution of patient conditions and the task of increasing a database of known record of cases that is necessary for training and testing expert algorithms used by processing means with CAD functionalities 6 and improving their performances in time and by the use by means of a constant learning. As an alternative or in combination, it is possible to provide also a reading/writing unit 8 of an external portable memory, such as a tape, a floppy-disk, a writeble or rewritable CD or DVD or a so called smart card wherein data of each examination for each patient are stored together with other data obtained from other examinations.

Between the portion of the apparatus for detecting MRI images and means for processing images and/or corresponding image data with CAD functionalities there is provided a unit generating and/or setting parameters and image acquisition sequences in nuclear magnetic resonance that is indicated by 9 that by means of a feedback line allows to obtain an adaptation and/or a direct optimization between means acquiring images and means processing them, such to set at best means acquiring images with reference to optimal requirements of processing means.

Processing means with CAD functionalities are composed of a plurality of software tools or modules that can be independently addressed one with respect to the other and that can be interfaced one with the other in any combinations depending on operations to which image data and/or resonance signals are desired to be subjected in order to obtain parameters indicating the orthopedic disease and its evolution state.

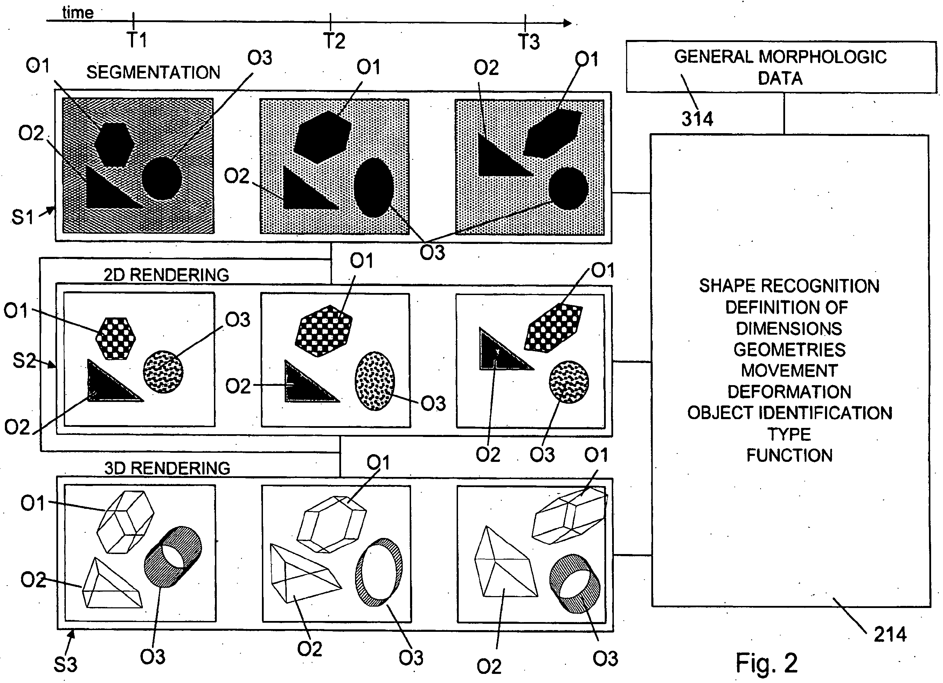

As shown in figure 2 by means of a segmentation module image data and/or corresponding pixels or voxels are divided in subsets, each of which is relevant to a specific object having its own identity independent of sets of image data and/or of pixels or voxels and each of which subsets of image data, pixels or voxels is the reproduction in the image of an independent or individual object being part of the body part or area under examination.

Segmentation allows to mainly define subsets of pixels or voxels of images that are in regions or volumes corresponding to the reproduction in the image of the real object. Once said subsets have been defined it is possible to transform each subset in a virtual object 01, 02, 03 therefore having its functional or semantic unit and that is the image of a real object comprised in the plane or volume of which the image has been acquired.

Therefore in each image acquired in different time moments indicated by T1, T2, T3 it is possible to recognize the object and by the comparison with one or more preceding images it is possible to determine the behaviour in time of each object as regards the position, orientation, shape and dimension and their geometry.

Once objects that can be composed of different types of tissues and/or components of a specific anatomical structure, and the behaviour in time thereof have been defined as said above, it is possible to provide the generation of a virtual image a kind of virtual copy of the real world wherein objects and behaviours are further highlighted by means of rendering, morphing, smoothing processes and other methods generating virtual realities.

In Figure 2 processes for recognizing shapes, for determining dimensions, geometries, the movement, orientation and shape changes, as well as the identification of real objects reproduced by virtual objects with reference to the kind and the task of these real objects are indicated by a subset 214, while the latter is interfaced with a further subset 314 providing morphologic and dimension data typical of real objects that can be compared with the ones determined in images.

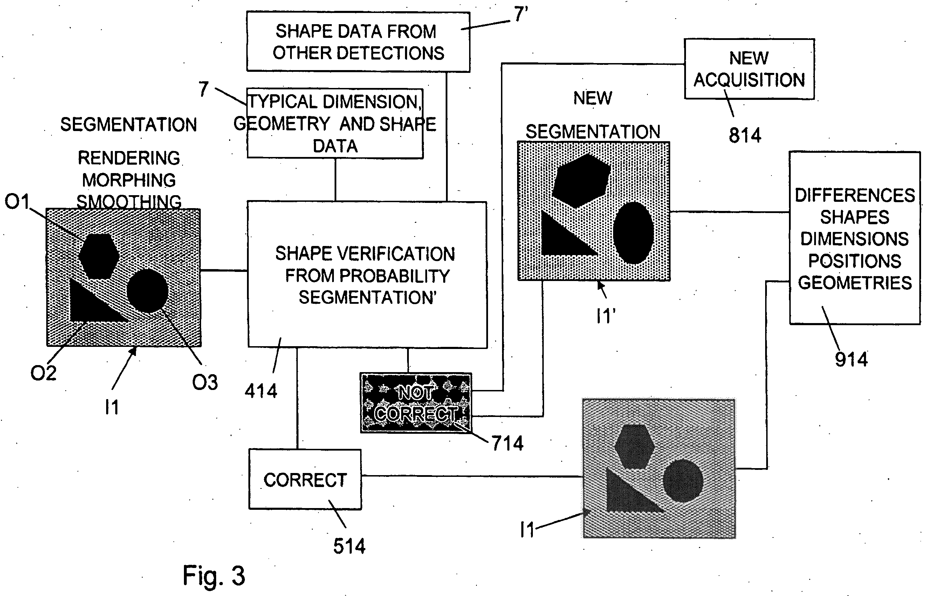

Figure 3 shows a further system for verifying the segmentation and the generation of renderized images as regards the compatibility of the morphology and dimensions and/or geometries of virtual objects identified in images with the morphology and/or dimensions and/or geometries typical of corresponding real ones possibly also with reference to morphologic, dimension and geometric changes caused by condition changes as in the present case by the presence of an othopedic disease.

In this case the image segmented and possibly further subjected to reconstruction by rendering possibly in combination with morphing or smoothing treatments, is analysed with reference to the shape and dimensions of objects 01, 02, 03 identified in said image I1 and possibly also of topologic and dimension relations of said objects one with respect to the other in a verification unit 414. To this unit 414 there are provided morphologic and/or dimension and/or geometric data typical of objects considered as to correspond to the ones reproduced in the image I1 and indicated by 01, 02, 03. Such data can be relevant both to an average value and/or to a range included between minimum and maximum values. Moreover typical data can also consider values that are not standard and corresponding to typical pathologic conditions. Processing means can comprise such data inside the database of clinical cases stored for example in the memory or memory area 7 as indicated in figure 1.

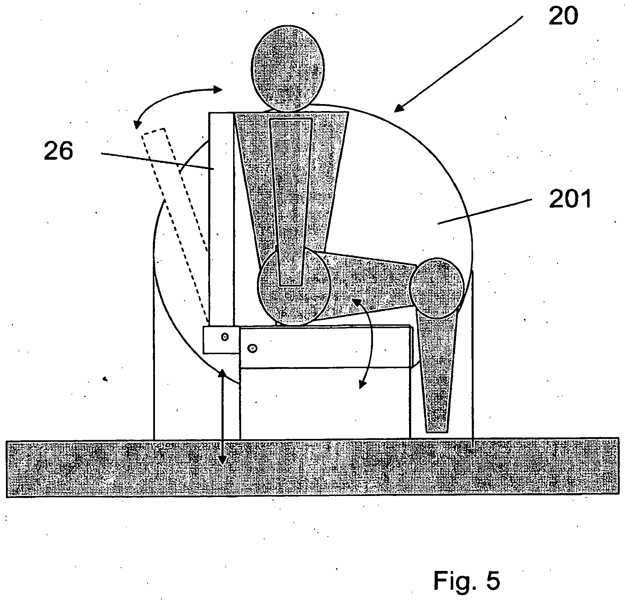

Moreover it is possible to make the comparison with morphologic, topologic, dimension and geometric data obtained by other measuring or analysing methods, such as by means of other image acquiring means different from the magnetic resonance such as ultrasound, radiologic means etc. said data being also included in the database of clinical cases and being stored in a dedicated memory area indicated by 7' in figure 5.

When the result of the verification system denotes that morphologic and/or dimension and/or geometric and/or topologic data of objects obtained from corresponding virtual objects are compatible with corresponding typical data it is possible to go on as indicated by the box 514 and by the image I1 and in this case for example it is possible to determine shape dimension and position differences of real objects determined by the corresponding virtual objects with respect to corresponding shape, dimension, geometry and position data of the same real objects as provided by the database of clinical cases 7 and 7' as indicated by the function box 914. Moreoever these differences can be used as a standard criterion for defining the existence of a pathologic condition considering the orthopedic point of view and/or for evaluating the evolution degree of the disease if it is present. It is possible to define different numerical comparison parameters according to different comparison functions and constituting numerical values. The comparison of these numerical values of comparison parameters with predetermined threshold values empirically defined on the basis of known clinical cases already allow to define a diagnostic indication.

When the verification unit 414 establishes that there is no compatibility between morphology and/or dimensions and/or geometry and/or position of real objects determined by virtual objects with respect to shape, dimensions and positions of real objects obtained by the database of clinical cases, so image data I1, segmented and/or further renderized and/or possibly subjected also to morphing and/or smoothing are considered as wrong ones 614 and it is possible both to repeat the segmentation and/or rendering process and/or possibly the morphing and/or smoothing process as indicated by the image I1' or even to provide a new acquisition of the image from the body under examination as indicated by 914.

However it is to be noted how all function boxes 414, 814, 914 are composed of program modules that are executed or can be executed upon call-up from the central processing unit and resident in a memory thereof or can be loaded in said memory.

As regards measures provided to be taken from image data and considered as measures indicating the presence or absence of an orthopedic disease and/or the evolution degree thereof the system according to the invention provides to use the segmentation process in combination with the rendering process in turn combined with further morphing and/or smoothing processes in order to highlight changes to the backbone structure between the supine position ahd the one under load. The image processing process allows to define conditions of main structures of a specific anatomical region as regards both geometry and contrast: it is also possible to process images by a simple analysis regarding RM contrast by means of which it is possible to automatically highlight pathologic areas.

A particular not limitative application of the invention refers to the diagnostic examination of the backbone. Like this examination the ones relevant to other anatomical regions are carried out.

Geometric parameters related to reciprocal space position of objects or structures considered in the supine position of the patient, in the upright position, and possibly in intermediate positions wherein the backbone is subjected to the natural load are defined by means of processing means for main structures of the backbone, particularly vertebrae, intervetebral discs and spinal canal for the lumbosacral and cervical portion in order to highlight by the comparison of dimension and/or geometric and/or morphologic data of the cartilage determined from image data of the patient under examination with dimension and/or morphologic data relevant to a plurality of known clinical cases included in a database of clinical cases. The comparison can be made by different functions and it provides information about differences and identities of dimensions, geometry and/or morphology of various structures of the backbone in the case under examination as regards dimensions, geometry, and/or morphology of various structures of the backbone of one or more known clinical cases, and so it provides a numerical quantitative indication about the presence/absence of an orthopedic disease and/or an indication of the evolution condition of said orthopedic disease.

Processing means provide one or more parameters just describing some quantities, particularly geometries or geometric shapes, that are considered to be valid for revealing the presence of the orthopedic disease and/or for determining the evolution condition of the orthopedic disease. It is also possible to combine such parameters with one or more further parameters that have been determined with other kinds of examinations and/or that are about the patient history or personal data and/or pathologic conditions according to previous examinations.

A variant provides individual parameters to have a different importance in determining the diagnostic indication.

A mode for determining the presence of the orthopedic disease and/or the evolution stage of such disease according to a very simplified embodiment is the simple comparison for said parameters with threshold numerical values.

In this case each parameter can be individually evaluated or it is possible to consider said parameters as being part of a vector of pathologic conditions and so to provide an overall evaluation of measured parameters and of threshold ones in the form of a comparison of the length of the corresponding vector whose components are composed of measured parameters for one of the vectors and of threshold values for said parameters for the comparison one.

For determining the presence of the orthopedic disease and/or the evolution condition thereof it is also possible to use other statistic systems evaluating parameters or any subcombination thereof.

Finally a particularly developped embodiment provides numerical parameters to be input values of a classification algorithm or a predictive algorithm such as for example an artificial neural network.

In this case the database of clinical cases and particularly of the specific patient are used for carrying out the training and testing step of the neural network.

It is interesting to consider the fact that the general database can lack in all or some specific data of the patient and so the network can be in an intermediate learning condition, while the learning ends during the step examinating the patient by loading the personal clinical database of the patient and by carrying out a further training and testing step by this database. Clinical data of the patient relevant to other preceding examinations both of the same type and of the different type can be stored in a storing movable medium such as a chip-card also known as smart card or the like that is read by the system at the imminence of an examination session by a suitable reader.

Therefore the system according to the present invention has to allow the storing of data obtained by above described methods during different examinations made on each patient.

The personal clinical database of the patient allows also to evaluate in a precise, economic, and rapid way the evolution of the disease both with a theraphy and without it.

From the above it is clear that the system according to the present invention allows to measure functional parameters intended to highlight the state of the orthopedic disease and to allow its follow-up during the theraphy and specifically these method consist in means for processing images for defining geometric parameters that highlight in a better way the presence of diseases and/or the comparative analysis of the contrast of RM images obtained at different stages of the disease. As regards the backbone the invention provides the segmentation of vertebrae and or disc structures intended to highlight the pathologic condition and the evolution during the theraphy, as well as the analysis regarding RM contrast aiming at highlighting pathologic regions in the more automatic possible way. At last the invention provides also means for automatically or semi-automatically highlithing possible restrictions of the spinal canal or the instability of vertebrae, by means of a depth clinical research activity.

It is also important to consider the fact that the method according to the present invention allows to deduce diagnosis suppositions or however to provide the medical staff with widening/highlighting cues and not with an automatic definitive diagnosis by the comparison with quantitative parameters measured during the occuring examination.

In order to allow to carry out the method described above it is necessary to develop a dedicated MRI system for applications in the orthopedic field allowing the implementation of high resolution three-dimensional methods.

With a particular reference to the making of means for acquiring MRI images, according to a preferrd embodiment of the invention they are composed of a scanner with a magnetic structure having permanent magnets, preferably made of neodymium. The field direction is provided as transversal to the magnet axis. The magnetic field is provided to have the following characteristics:
Static field intensity: 0.21 T ± .7 mT (f0: 10.22 MHz ± 300 KHz)
Homogeneity: < ± 4 ppm FWHM on 250 mm DSV
Shimming system: passive
Possibility of modifying the magnetic field: ±1 mT (± 45 KHz).
Dispersed field (line at 0.5 mT): within 2 meters from magnetic unit centre
Magnetic shielding: not necessary
Other parameters are the following:
   Gantry opening: usable width at least 38 cm; height to be defined
   Space resolution: up to 0.4 mm
   View: 250 mm x 250 mm

Advantageously there is provided the use of pole pieces for a considerable Eddy current reduction and of receiving coils with Dual Phased Array technology.

Gradient system provides a maximum intensity: included between ± 15 mT/m and ± 20 mT/m a rise time: 0.5 ms from 0 to +15 mT/m at 99% and a Linearity : ± 5 % on 140 mm DSV.

In combination there are also provided RF electromagnetic shielding means.

As regards electronic/information characteristics of the tomograph there is provided:
- Operating system working in Windows environment, implemented on PC hardware;
- Native implementation of DICOM protocol;
- Software application "operator interface" uniform for standard PC working station of the tomograph and for remote PC working station allowing to carry out tele-diagnosis;
- Software application for transmitting texts and image in real time, even during the examination;
- Devices intended to allow automatic adjustments and calibrations, also remote ones (teleservice).

As regards functional-applicative characteristics:
- DPA coils (or openable linear) for the cervical column
- DPA coils (or with more channels) for the lumbosacral column
- set of 3D acquisition sequences with different contrast (T1, T2) both in echo gradient and spin-echo, aiming at developping methods for the 3D segmentation of organs under interest
- 2D multi-slice, turbo, with Fst SE reconstruction and acquisition mechanism ( sequences SET2, ME and STIR) with high resolution arrays up to 512x512.
- 2D Real-time acquisitions for dynamic studies
- Fat suppression with DIXON method or the like.

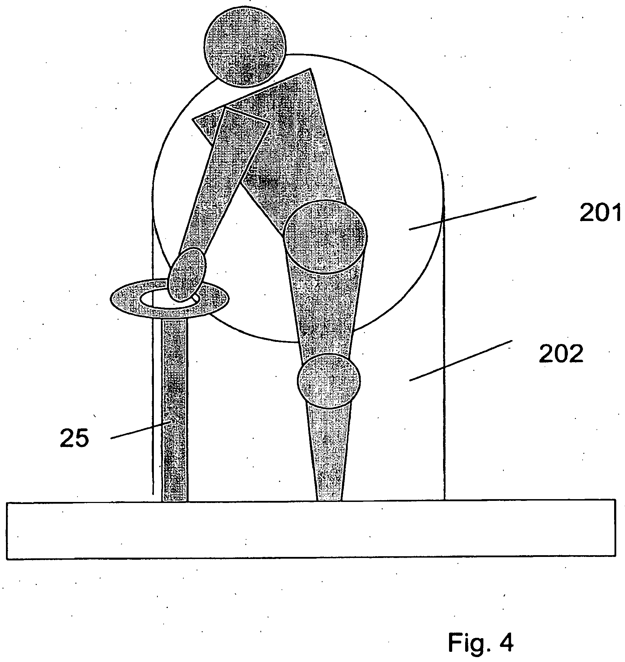

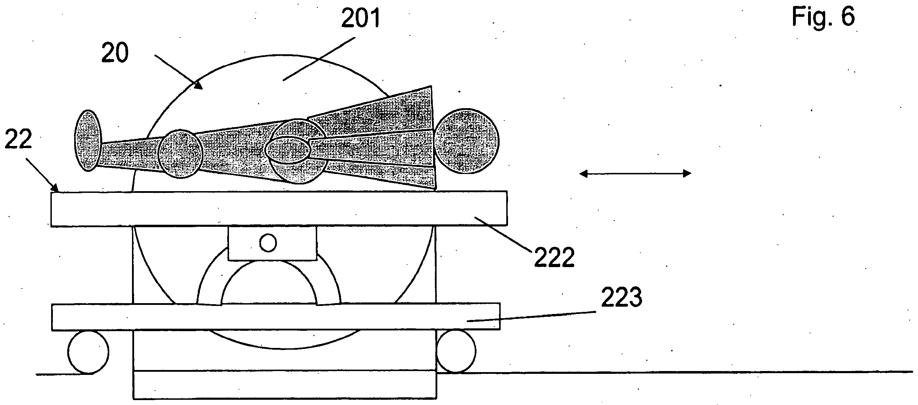

With reference to figures 4 to 6, a not limitative example of an apparatus for acquiring images in the orthopedic field, particularly of the backbone anatomical region, by nuclear magnetic resonance, comprises a magnetic structure generally indicated by 20 intended for generating a static magnetic field in an horizontal plane between the two poles 201. Said magnetic structure defines a cavity having an opening for the introduction of the body part under examination or through which the patient can enter by a simple self-deambulation or being transported on a movable supporting means such as an examination table, a wheelchair or a similar device such that the body part to be examined is placed inside the detecting cavity.

Particularly the MRI apparatus allows to house the patient for methods examining the muscle-skeletal apparatus, including the backbone for the lumbosacral and cervical portion, such to allow the acquisition of images of the anatomical region of interest at resting condition or under load.

The two opposite poles 201 generating the magnetic field constitute or are borne by two opposite vertical walls that are spaced apart such to form said space housing the patient therebetween and at least at an end side at least an entering opening. Means exciting the body under examination or a part thereof upon the emission of resonance signals are integrated in or are borne by said walls or are combined with said poles 201.

The wall 202 connecting the two poles 201 and/or the walls supporting said two poles 201 can be the bearing base of the magnetic structure 20 and the trampling surface for the patient when he is inside the housing space or it can be in the vertical position therefore the magnetic structure 20 is composed of three closed vertical walls constituted by poles 201 or by the associated supporting walls and by the wall 202, whereas the trampling surface is composed of the floor itself or of a further base. The fact that the wall 202 constitutes the top wall can be assumed the structure being overturned at 180°. Instead of a magnet with a C or U shaped section as the one shown it is possible to provide magnetic structures with a closed annular shape that are opened at one side or two sides transversal to the axis of the annular structure, magnets wherein the two poles 201 are horizontal or substantially horozontal and are kept spaced apart by means of two or more columns arranged along the perimeter of said poles.

Obviously the MRI apparatus for detecting images by nuclear magnetic resonance has usual and known transmitting coils and gradient coils and processing and controlling electronics. These parts are not shown in details since are generally known and are not the object of the present invention.

The apparatus comprises means for positioning and/or leaning and/or retaining and/or supporting the body of the patient or the body part under examination that are provided as to be assembled and disassembled and/or moved inside the cavity housing the patient or the body part under examination for comfortably positioning the patient and for accurately optimizing the signal to noise ratio.

Said means can be also possibly combined one with the other.

In a preferred embodiment the configuration of the magnetic structure 20 allows to rotate the magnet together with means for positioning and/or leaning and/or retaining and/or supporting the body of the patient or the body part under examination such that the patient can be comfortably housed in the detection cavity for example lay on an examination table first in a supine position and then, after the rotation, in the upright position and/or in intermediate positions, wherein orthopedic structures such as the backbone are subjected to the natural load.

Means for positioning and/or leaning and/or retaining and/or supporting the body of then patient or the body part under examination can comprise housing and/or passage guides for different devices such as means receiving resonance signals.

Particularly said positioning means can be composed of a supporting examination table 22 comprising an horizontal supporting plane 222 for the patient that is intended to be laid in the supine position on said plane. The supporting plane is borne by a bearing structure 223 having a certain thickness and in which a chamber housing the receiving coil 23 can be obtained.

It is possible to provide receiving coils and/or means for receiving resonance signals 23 to be integrated in walls 201 constituting or bearing poles. Receiving coils can be housed in said walls or can be borne thereby and can be integrated in poles. In combination or as an alternative means receiving resonance signals 23 can be movably fastened directly on the body under examination or on a part thereof. Such means receiving resonance signals, that is receiving coils 23, can be removably fastened directly on the body of the patient and in the region of one or more separate anatomical regions to be examined.

Such as in the case of the examination table 22 it is possible to provide receiving coils 23 to be integrated with means for positioning and/or leaning and/or retaining and/or supporting the body of the patient or the body part under examination.

The apparatus for acquiring MRI images can be oriented in an integrated and integral way with means for positioning and/or leaning and/or retaining and/or supporting the body of the patient or the body part under examination such to allow the examination of the anatomical region of interest, for example the backbone, both in the laid position and in the upright position and to detect images of the anatomical region of interest with anatomical structures in resting conditions and under load.

The examination table 22 and/or other similar devices can also have means for adjusting the height of the supporting plane 222 for the patient and/or the tilting about one or more axis such that the patient is moved according to different orientations in the space. Particularly it is possible to adjust the tilting of the supporting plane 222 such that the examination table 22 is brought from an horizontal position or in a resting condition of muscle skeletal structures of orthopedic interest to a substantially upright position or to a loaded position of said structures, stopping the examination table 22 according to different tilting degrees such to acquire MRI images of the anatomical region of the backbone or of other orthopedic regions according to different loading degrees, till completely loading the anatomical region ofn interest when the examination table is brought in a complete vertical position. Movable locking means allow to release the plane and so to move in a predetermined tilting position, then movable locking means being again operated for retaining the examination table in the selected tilted position. Instead of the examination table it is possible to provide any other device such as for example a chair and/or a chair that can become an examination table.

As an alternative to the examination table it is possible to provide a chair element 26 that can be mounted or placed in a movable way and in different positions inside the examination cavity. the chair 26 has means for adjusting the height of the seat that can be tilted according to at least an axis oriented in the direction of the magnetic field and it can be provided with at least a back part that can be tilted according to at least an axis oriented in the direction of the magnetic field.

The patient can take any positions or arrangements inside the detecting cacity, for example he can be forwardly or rearwardly bent such as shown in figure 4 or the patient can be in his upright position with the axis joining the shoulders substantially perpendicular to poles 201 and/or parallel to the magnetic field therebetween. In order to facilitate the fact of taking some positions particularly difficult from the balance point of view and in order to help the patient to keep the region of interest in the resting condition and/or under stress during the examination, there are provided means for positioning and/or leaning and/or retaining and/or supporting the body of the patient or the body part under examination.

According to an embodiment if the orthopedic part to be examined is comprised in the anatomical region of legs and/or of the backbone, particularly the lumbosacral portion, said positioning means can be composed of one or more footrests having different heights and/or having adjustable heights, that can be movably mounted and in different positions on the base 202 or on the trampling plane of the magnetic structure inside the detecting cavity.

Braking means, and/or stopping means such as abutments, movable and/or adjustable limit stops can allow to temporarily stop the footrest inside the housing cavity in one or more predetermined positions corresponsing to one or more predetermined imaging positions in one or more different anatomical regions. Ideal homogeneity conditions of the static magnetic field are present in the centered part of the total volume of the housing cavity. Therefore in order to acquire diagnostic images of an anatomical region and to reduce dimension of MRI apparati it is necessary for said region to coincide with the partial volume wherein optimal homogeneity conditions of the magnetic field are present that is the so called imaging volume.

Said footrests can help the patient in keeping a specific position or can force the patient to keep a specific unintentional position such that anatomical structures of interest are stimulated. Since the method object of the present invention allows to carry out morpho-functional analyses in the orthopedic field said means for positioning and/or leaning and/or retaining and/or supporting the body of the patient or the body part under examination can help the patient to make a specific movement during the step acquiring images such that an image sequence is obtained that will be used to create a film of the movement that has been made.

A further positioning and/or leaning and/or retaining and/or supporting means that can be provided individually or in combination with other positioning means is composed of one or more holding handles 25 or of other holding means provided in a firm or movable way and fastenable in different positions inside the detecting cavity.

Handles 25 can be provided in combination with footrests in such a position to allow the patient to keep a position stimulating the knee and/or other orthopedic regions involved in the rised movement without an excessive effort.

Holding handles 42 can be composed of any kind of bracket, rod, posts or the like and can be mounted in any positions inside the magnetic strucutre 20 such to position or help the positioning of the patient or of the body part under examination, such as for example joints of the upper part of the body or the cervical portion of the backbone during the acquisition of images or of image sequence under resting condition of the anatomical structure to be examined or under stimulating conditions and/or under load: the patient is asked to make a predetermined movement such as a flat and/or uphill walking movement during the acquisition of MRI images, a base like a tapis roulant being provided that can be tilted and/or movement going on or down by steps and/or a seating movement or a movement passing from the seated position to the upright one or a forwardly or rearwardly bending movement and/or laterally while during said movements a time sequence of MRI image acquisitions is carried out of at least one or more anatomical regions of orthiopedic interest.

Naturally the invention is not to be intended as limiting described and shown embodiments, but it can be widely modifed, above all from the constructive point of view, without departing from the information principle mentioned above and claimed below.

## Claims

1. Apparatus for determining indications helping the diagnosis of orthopedic diseases comprising a section for detecting and acquiring signals from a body under examination or from a part thereof further comprising a detecting section and a section for acquiring images by nuclear magnetic resonance (NMR), wherein the detecting section is a unit for detecting
images by nuclear magnetic resonance (NMR) and the section for acquiring images by NMR is a unit for acquiring
images by NMR having MRI scanning means that are shaped and have such a dimension that allow the acquisition of MRI images from the anatomical region of orthopedical interest in the region of the backbone, particularly of the backbone in the lumbosacral and/or cervical portion and said unit for detecting images by NMR is integrated in a section for processing acquired images, i.e. the processing section, as regards image data and/or resonance signals, so that said unit for acquiring images by nuclear magnetic resonance and said section for processing acquired images are grouped together in a single apparatus, which processing section defines, from image data and/or resonance signals, values of one or more different numerical parameters as geometric parameters, concerning dimensions and/or shape and/or volume and/or geometries of the muscle skeletal structures of the backbone, indicating the presence or absence of an orthopedic disease and/or a measure of the evolution condition of said orthopedic disease, whose processing functionalities being carried out under the "on line" mode by the MRI scan, i.e. immediately after having acquired image data and the acquisition functionalities of said section for acquiring images being guided by the processing section itself by means of a feedback line, wherein the processing section comprises a sub-section with means for determining the geometric parameters of main muscle skeletal structures of orthopedic interest of the backbone, particularly of vertebrae, disc structures and/or spinal canal, said geometric parameters being detected at different tilting degrees of the backbone, particularly with the backbone in a substantially horizontal position, i.e. at resting condition, and/or in a substantially vertical position, i.e. under load, and/or in intermediate positions between the horizontal and vertical position of the backbone.

2. Apparatus according to claim 1, **characterized in that** the processing section comprises a sub-section with means for determining the geometric parameters concerning the reciprocal space position of main structures of the backbone, particularly of vertebrae, disc structures and/or spinal canal in a substantially horizontal position, i.e. at resting condition, and/or in a substantially vertical position, i.e. under load, and/or in intermediate positions between the horizontal and vertical position of the backbone.

3. Apparatus according to one or more of the preceding claims, **characterized in that** the processing section comprises a sub-section with means for comparing said parameters with a reference value for discriminating the presence/absence of a pathologic condition and/or for the comparison with a reference scale for determining a parameter indicating the evolution degree of the pathologic condition, which reference value for discriminating the presence /absence of the pathologic condition and/or which reference scale for determining the evolution degree of the pathologic condition are included in a database of known clinical cases.

4. Apparatus according to one or more of the preceding claims, **characterized in that** the processing section comprises a sub-section with means determining numerical values of said pathologic condition of muscle skeletal structures of orthopedic interest such as joints or other ones and particularly of backbone structures, both regarding the presence/absence of it and the evolution condition of the disease by means of the comparative analysis of the contrast of MR images such as the detection of maps in T1 or T2 and/or analysis of images by means of suitable contrast media, obtained at different stages of the pathologic condition from images relevant to patients being part of the database of known clinical cases and/or from images relevant to previous indagations carried out on the same patient.

5. Apparatus according to one or more of the preceding claims, **characterized in that** the processing section comprises a sub-section with means for determining numerical values of said pathologic condition of backbone structures both regarding the presence/absence of it and the evolution condition of the disease by processing image data by means of classification and/or predictive algorithms, which have been trained, by training and testing, by means of image data regarding the backbone of known clinical cases included in a database of known clinical cases.

6. Apparatus according to one or more of the preceding claims, **characterized in that** the processing section comprises a sub-section determining the pathologic state of muscle skeletal structures of orthopedic interest and of the backbone structures with means for segmenting images, such as rendering means possibly combined with morphing and/or smoothing means, for determining subsets of image data and/or pixels and/or voxels and for identifying real objects represented in the image by said subsets of image data and/or pixels and/or voxels, the subset of pixels or voxels or image data being defined representing the main structures, regarding the anatomical region of the backbone, particularly vertebrae and disc structures and dimensions and/or shape and/or geometries of said structures being determined.

7. Apparatus according to claim 6, **characterized in that** the sub-section determining dimensions, geometry and/or morphology of orthopedic structures of the anatomical region under examination, in the case of the anatomical region of the backbone, particularly of vertebrae and intervertebral discs comprises means for comparing dimension, geometric and/or morphologic data of main structures of the region under examination with dimension, geometric and morphologic data of a plurality of known clinical cases included in a database of clinical cases which comparison provides information about the differences and indentities of dimensions and/or geometry and/or morphology of the main structures of the region under examination with respect to dimensions and/or geometries and/or morphology of said structures of one or more known clinical cases, and therefore it provides an indication about the presence /absence of an orthopedic disease and/or an indication about the evolution condition of said orthopedic disease, for example, such to highlight possible restrictions of the spinal canal and/or the instability of vertebrae in the case of the anatomical region of the backbone.

8. Apparatus according to claim 6, **characterized in that** it comprises means for verifying the reliability of the identification of the subset of pixels or voxels or image data representing in the image or in the set of image data a real object and in the case of the backbone anatomical region, particularly vertebrae, disc structures and/or the spinal canal which verification means comprise a database of dimension, geometric and/or volume and/or morphologic configuration data that is of typical shapes of main structures of the region under examination in specific conditions of absence or presence of a disease and/or a specific evolution degree of the disease whereas dimensions, geometry and/or volume and/or the morphology of structures under examination determined by sub-sections of means processing images of a patient under examination are compared with said typical dimension, geometric and/or volume data and/or typical morphologies for said structures that are in the form of average values and/or a range of average values of dimension, geometric and/or volume and/or morphologic differences a first difference maximum threshold being determined, whose exceeding makes dimension, geometric and/or volume and/or morpgologic data to be considered as to be incompatible and unreliable ones, verification means being provided with a sub-section signalling or requiring the repetition of the process for determining dimensions and/or geometry and/or volume and/or morphologic characteritics and/or of the acquisition of MRI image or images or said verifying means are provided with a sub-section automatically commanding the repetition of the process for determining dimensions and/or geometry and/or volume and/or morphologic characteristics and/or the acquisition of MRI image or images.

9. Apparatus according to one or more of the preceding claims, **characterized in that** the detecting section, particularly the unit detecting images by nuclear magnetic resonance is connected to the section processing acquired images by means of the feed-back line.

10. Apparatus according to one or more of the preceding claims, **characterized in that** the processing section comprises means for analysing acquired images as regards predetermined quality parameters of said image data and/or resonance signals and means for automatically changing acquisition settings and/or acquisition parameters of image data that are controlled by said means analysing image data on the basis of quality parameters of image data and/or of resonance signals from which they are determined.

11. Apparatus according to one or more of the preceding claims **characterized in that** the processing section provides a sub-section for determining the pathologic state and/or the presence of damages to orthopedic structures comprising classificating and/or predicting means such as for example a classificating and/or predictive algorithm that are trained by data of a database of known clinical cases and to which there are provided parameters indicating the presence/absence of a disease and/or the evolution degree of the disease as input data determined by sub-sections determining dimensions, geometries and/or the morphology of muscle skeletal structures of orthopedic interest and particularly the structures of the backbone anatomical region and possibly further data obtained by different examinations and/or personal data or the medical history of the patient.

12. Apparatus according to one or more of the preceding claims, **characterized in that** it is provided with means for storing known clinical cases and/or diagnostic images and/or processing data and/or diagnostic data of previous examinations relevant to an orthopedic disease and possibly also other diseases for each patient, acquired in different time moments even at relatively long time intervals, such as days, months or years, said data being used to determine at least one or more reference values for discriminating the presence/absence of a disease and/or a scale of numerical values for different evolution degrees of the disease, and said values being compared with dimension, geometric and morphologic values of structures of the backbone for determining the evolution, i.e. the follow-up, of a disease and the kind of intervention to be carried out and/or to verify the efficacy of a therapy in progress.

13. Apparatus according to claim 1, **characterized in that** the unit for detecting images by nuclear magnetic resonance is composed of a magnetic structure defining a cavity having an opening for the introduction of the body part under examination or through which the patient can enter by a simple self-deambulation or being transported on a movable supporting means such as an examination table, a wheelchair or a similar device such that the body part to be examined is placed inside the detecting cavity, particularly portions of the muscle-skeletal apparatus, such as the backbone for the lumbosacral and cervical portion, such to allow the acquisition of images of the anatomical region of interest at resting condition or under load.

14. Apparatus according to claim 12 **characterized in that** it comprises means for positioning and/or leaning and/or retaining and/or supporting the body of the patient or the body part under examination that are provided as to be assembled and disassembled and/or moved inside the cavity housing the patient or the body part under examination for comfortably positioning the patient and for accurately optimizing the signal to noise ratio.

15. Apparatus according to claims 13, 14 **characterized in that** the magnet and the means for positioning and/or leaning and/or retaining and/or supporting the body of the patient or the body part under examination rotate together such that the patient can be comfortably housed in the detection cavity for example lay on an examination table first in a supine position and then, after the rotation, in the upright position and/or in intermediate positions, wherein orthopedic structures such as the backbone are subjected to the natural load.

16. Apparatus according to claims 13 to 15, **characterized in that** the means for positioning and/or leaning and/or retaining and/or supporting the body of the patient or the body part under examination comprise housing and/or passage guides for different devices such as means receiving resonance signals.

17. Apparatus according to claims 13 to 16, **characterized in that** the means (23) receiving resonance signals can be movably fastened directly on the body under examination or on a part thereof and/or are integrated in walls (202) constituting or bearing poles (201).

18. Apparatus according to claims 13 to 17, **characterized in that** the means for positioning and/or leaning and/or retaining and/or supporting the body of the patient or the body part under examination are composed of an examination table (22) or the like provided with means for adjusting the height of the supporting plane (222) for the patient and/or the tilting about one or more axis such that the patient is moved according to different orientations in the space, particularly said means allows to adjust the tilting of the supporting plane (222) such that the examination table (22) is brought from an horizontal position to a substantially upright position, stopping the examination table (22) according to different tilting degrees such to acquire MRI images of the anatomical region of the backbone or of other orthopedic regions according to different loading degrees, till completely loading the anatomical region of interest when the examination table is brought in a complete vertical position

19. Apparatus according to claims 13 to 18, **characterized in that** the means for positioning and/or leaning and/or retaining and/or supporting the body of the patient or the body part under examination are composed of chair element (26) that can be mounted or placed in a movable way and in different positions inside the examination cavity and it has means for adjusting the height of the seat that can be tilted according to at least an axis oriented in the direction of the magnetic field and at least a back part that can be tilted according to at least an axis oriented in the direction of the magnetic field.

20. Apparatus according to claims 13 to 19, **characterized in that** the means for positioning and/or leaning and/or retaining and/or supporting the body of the patient or the body part under examination are composed of one or more footrests having different heights and/or having adjustable heights, that can be movably mounted and in different positions on the base (202) or on the trampling plane of the magnetic structure inside the detecting cavity.

21. Apparatus according to claims 13 to 20, **characterized in that** said means for positioning and/or leaning and/or retaining and/or supporting the body of the patient or the body part under examination are provided individually or in combination with other positioning means composed of one or more holding handles (25) or of other holding means provided in a firm or movable way and fastenable in different positions inside the detecting cavity.

## Patentansprüche

1. Einrichtung zur Bestimmung von Indikationen zur Unterstützung der Diagnose von orthopädischen Krankheiten, aufweisend einen Abschnitt zur Erkennung und Erfassung von Signalen von einem untersuchten Körper oder von einem Teil von diesem, ferner aufweisend einen Detektionsabschnitt und einen Abschnitt zur Erfassung von Bildern durch kernmagnetische Resonanz (Nuclear Magnetic Resonance, NMR), wobei der Detektionsabschnitt eine Einheit zur Erkennung von Bildern durch kernmagnetische Resonanz (NMR) ist und der Abschnitt zur Erfassung von Bildern durch NMR eine Einheit zur Erfassung von Bildern durch NMR mit MRT-Scanmitteln ist, die so geformt und bemessen sind, dass sie die Erfassung von MRT-Bildern von dem anatomischen Bereich von orthopädischem Interesse im Bereich der Wirbelsäule, insbesondere der Wirbelsäule im lumbosakralen und/oder zervikalen Teil, ermöglichen, und wobei die Einheit zur Erkennung von Bildern durch NMR in einen Abschnitt zur Verarbeitung von erfassten Bildern, d. h. den Verarbeitungsabschnitt, betreffend Bilddaten und/oder Resonanzsignale, integriert ist, so dass die Einheit zur Erfassung von Bildern durch kernmagnetische Resonanz und der Abschnitt zur Verarbeitung von erfassten Bildern in einer einzelnen Einrichtung zusammengefasst sind, wobei der Verarbeitungsabschnitt anhand von Bilddaten und/oder Resonanzsignalen Werte von einem oder mehr verschiedenen numerischen Parametern als geometrische Parameter definiert, betreffend Abmessungen und/oder Form und/oder Volumen und/oder Geometrien der Muskel-Skelett-Strukturen der Wirbelsäule, die das Vorliegen oder Fehlen einer orthopädischen Krankheit und/oder ein Maß des Entwicklungszustands der orthopädischen Krankheit anzeigen, wobei dessen Verarbeitungsfunktionalitäten unter dem "Online-Modus" durch den MRT-Scan ausgeführt werden, d. h. unmittelbar nachdem Bilddaten erfasst wurden, und wobei die Erfassungsfunktionalitäten des Abschnitts zur Erfassung von Bildern mittels einer Rückkopplungsleitung durch den Verarbeitungsabschnitt selbst geführt werden, wobei der Verarbeitungsabschnitt einen Unterabschnitt mit Mitteln zur Bestimmung der geometrischen Parameter von Haupt-Muskel-Skelett-Strukturen der Wirbelsäule von orthopädischem Interesse, insbesondere der Wirbelknochen, der Bandscheibenstrukturen und/oder des Spinalkanals, aufweist, wobei die geometrischen Parameter bei unterschiedlichen Neigungsgraden der Wirbelsäule erkannt werden, insbesondere wobei sich die Wirbelsäule in einer im Wesentlichen horizontalen Position, d. h. in einem Ruhezustand, und/oder in einer im Wesentlichen vertikalen Position, d. h. unter Last, und/oder in Zwischenpositionen zwischen der horizontalen und der vertikalen Position der Wirbelsäule befindet.

2. Einrichtung nach Anspruch 1, **dadurch gekennzeichnet, dass** der Verarbeitungsabschnitt einen Unterabschnitt mit Mitteln zur Bestimmung der geometrischen Parameter betreffend die gegenseitige Raumposition von Hauptstrukturen der Wirbelsäule, insbesondere von Wirbelknochen, Bandscheibenstrukturen und/oder vom Spinalkanal, in einer im Wesentlichen horizontalen Position, d. h. im Ruhezustand, und/oder in einer im Wesentlichen vertikalen Position, d. h. unter Last, und/oder in Zwischenpositionen zwischen der horizontalen und der vertikalen Position der Wirbelsäule, aufweist.

3. Einrichtung nach einem oder mehreren der vorhergehenden Ansprüche, **dadurch gekennzeichnet, dass** der Verarbeitungsabschnitt einen Unterabschnitt mit Mitteln zum Vergleichen der Parameter mit einem Referenzwert zur Unterscheidung zwischen dem Vorliegen/Fehlen eines pathologischen Zustands und/oder zum Vergleichen mit einer Referenzskala zur Bestimmung eines Parameters, der den Entwicklungsgrad des pathologischen Zustands anzeigt, aufweist, wobei der Referenzwert zur Unterscheidung zwischen dem Vorliegen/Fehlen des pathologischen Zustands und/oder die Referenzskala zur Bestimmung des Entwicklungsgrads des pathologischen Zustands in einer Datenbank von bekannten klinischen Fällen enthalten sind.

4. Einrichtung nach einem oder mehreren der vorhergehenden Ansprüche, **dadurch gekennzeichnet, dass** der Verarbeitungsabschnitt einen Unterabschnitt mit Mitteln zur Bestimmung von numerischen Werten des pathologischen Zustands der Muskel-Skelett-Strukturen von orthopädischem Interesse aufweist, wie Gelenken oder anderen und insbesondere von Wirbelsäulenstrukturen, die jeweils das Vorliegen/Fehlen von diesem und den Entwicklungszustand der Krankheit durch die Vergleichsanalyse des Kontrastes von Bildern betreffen, wie die Detektion von Karten in T1 oder T2 und/oder die Analyse von Bildern durch geeignete Kontrastmittel, erhalten in verschiedenen Stadien des pathologischen Zustands anhand von Bildern, die Patienten betreffen, die Teil der Datenbank von bekannten klinischen Fällen sind, und/oder anhand von Bildern, die frühere Untersuchungen betreffen, die am selben Patient ausgeführt worden sind.

5. Einrichtung nach einem oder mehreren der vorhergehenden Ansprüche, **dadurch gekennzeichnet, dass** der Verarbeitungsabschnitt einen Unterabschnitt mit Mitteln zur Bestimmung von numerischen Werten des pathologischen Zustands von Wirbelsäulenstrukturen sowohl betreffend das Vorliegen/Fehlen von diesem als auch den Entwicklungszustand der Krankheit durch Verarbeitung von Bilddaten mittels Klassifizierung und/oder prädiktiven Algorithmen aufweist, die mittels Bilddaten betreffend die Wirbelsäule von bekannten klinischen Fällen, die in einer Datenbank von bekannten klinischen Fällen enthalten sind, durch Schulung und Prüfung geschult wurden.

6. Einrichtung nach einem oder mehreren der vorhergehenden Ansprüche, **dadurch gekennzeichnet, dass** der Verarbeitungsabschnitt einen Unterabschnitt aufweist, der den pathologischen Zustand von Muskel-Skelett-Strukturen von orthopädischem Interesse und der Wirbelsäulenstrukturen bestimmt, mit Mitteln zur Segmentierung von Bildern, wie Rendering-Mitteln, gegebenenfalls kombiniert mit Morphing- und/oder Glättungsmitteln, zur Bestimmung von Untersätzen von Bilddaten und/oder Pixeln und/oder Voxeln, und zur Erkennung von realen Objekten, die in dem Bild durch die Untersätze von Bilddaten und/oder Pixeln und/oder Voxeln dargestellt sind, wobei der Untersatz von Pixeln oder Voxeln oder Bilddaten als die Hauptstrukturen darstellend definiert sind, betreffend den anatomischen Bereich der Wirbelsäule, insbesondere von Wirbelknochen und Bandscheibenstrukturen und Größen und/oder die Form und/oder Geometrien der Strukturen, die bestimmt werden.

7. Einrichtung nach Anspruch 6, **dadurch gekennzeichnet, dass** der Unterabschnitt, der Größen, die Geometrie und/oder Morphologie von orthopädischen Strukturen des untersuchten anatomischen Bereichs bestimmt, im Fall des anatomischen Bereichs der Wirbelsäule, insbesondere von Wirbelknochen und Bandscheiben, Mittel zum Vergleichen von Größen-, geometrischen und/oder morphologischen Daten von Hauptstrukturen des untersuchten Bereichs mit Größen-, geometrischen und morphologischen Daten einer Vielzahl von bekannten klinischen Fällen, die in einer Datenbank von klinischen Fällen enthalten sind, aufweist, wobei der Vergleich Informationen über die Unterschiede und Identitäten von Größen und/oder der Geometrie und/oder der Morphologie der Hauptstrukturen des untersuchten Bereichs in Bezug auf Größen und/oder Geometrien und/oder die Morphologie der Strukturen von einem oder mehr bekannten klinischen Fällen liefert, und daher stellt sie eine Indikation über das Vorliegen/Fehlen einer orthopädischen Krankheit und/oder eine Indikation über den Entwicklungszustand der orthopädischen Krankheit bereit, zum Beispiel, um mögliche Einschränkungen des Spinalkanals und/oder die Instabilität von Wirbelknochen im Fall des anatomischen Bereichs der Wirbelsäule hervorzuheben.

8. Einrichtung nach Anspruch 6, **dadurch gekennzeichnet, dass** sie Mittel zur Überprüfung der Zuverlässigkeit der Identifizierung des Untersatzes von Pixeln oder Voxeln oder Bilddaten aufweist, die in dem Bild oder in dem Satz von Bilddaten ein reales Objekt darstellen, und im Fall des anatomischen Wirbelsäulenbereichs, insbesondere der Wirbelknochen, Bandscheibenstrukturen und/oder des Spinalkanals, wobei die Überprüfungsmittel eine Datenbank von Größen-, geometrischen und/oder Volumen- und/oder morphologischen Konfigurationsdaten aufweisen, die typische Formen von Hauptstrukturen des untersuchten Bereichs bei spezifischen Zuständen des Fehlens oder Vorliegens einer Krankheit und/oder eines spezifischen Entwicklungsgrads der Krankheit haben, während Größen, die Geometrie und/oder das Volumen und/oder die Morphologie von untersuchten Strukturen, die durch Unterabschnitte von Mitteln bestimmt werden, die Bilder von einem untersuchten Patient verarbeiten, mit den typischen Größen-, geometrischen und/oder Volumendaten und/oder typischen Morphologien für die Strukturen verglichen werden, die in Form von Durchschnittswerten und/oder eines Bereichs von Durchschnittswerten der Größen-, geometrischen und/oder Volumen- und/oder morphologischen Unterschiede vorliegen, wobei eine erste maximale Schwelle für Unterschiede bestimmt wird, wobei deren Überschreitung dazu führt, dass die Größen-, geometrischen und/oder Volumen- und/oder morphologischen Daten als inkompatible und unzuverlässige Daten angesehen werden, wobei Überprüfungsmittel mit einem Unterabschnitt vorgesehen sind, der die Wiederholung des Verfahrens zur Bestimmung von Größen und/oder der Geometrie und/oder des Volumens und/oder morphologischer Kennzeichen und/oder der Erfassung eines MRT-Bilds oder von MRT-Bildern meldet oder erfordert, oder wobei die Überprüfungsmittel mit einem Unterabschnitt versehen sind, der automatisch die Wiederholung des Verfahrens zur Bestimmung von Größen und/oder der Geometrie und/oder des Volumens und/oder von morphologischen Kennzeichen und/oder die Erfassung eines MRT-Bilds oder von MRT-Bildern befiehlt.

9. Einrichtung nach einem oder mehreren der vorhergehenden Ansprüche, **dadurch gekennzeichnet, dass** der Detektionsabschnitt, insbesondere die Einheit, die Bilder durch kernmagnetische Resonanz erkennt, mit dem Abschnitt verbunden ist, der erfasste Bilder durch die Rückkopplungsleitung verarbeitet.

10. Einrichtung nach einem oder mehreren der vorhergehenden Ansprüche, **dadurch gekennzeichnet, dass** der Verarbeitungsabschnitt Mittel zur Analyse von erfassten Bildern betreffend vorbestimmte Qualitätsparameter der Bilddaten und/oder Resonanzsignale und Mittel zur automatischen Änderung von Erfassungseinstellungen und/oder Erfassungsparametern von Bilddaten aufweist, die durch die Mittel zur Analyse von Bilddaten auf Grundlage von Qualitätsparametern von Bilddaten und/oder von Resonanzsignalen, anhand welcher sie bestimmt werden, gesteuert werden.

11. Einrichtung nach einem oder mehreren der vorhergehenden Ansprüche, **dadurch gekennzeichnet, dass** der Verarbeitungsabschnitt einen Unterabschnitt zur Bestimmung des pathologischen Zustands und/oder des Vorliegens von Schäden an orthopädischen Strukturen bereitstellt, aufweisend Klassifizierungs- und/oder Prädiktionsmittel, wie zum Beispiel einen Klassifizierungs- und/oder Prädiktionsalgorithmus, die durch Daten einer Datenbank von bekannten klinischen Fällen geschult werden und denen Parameter zur Verfügung gestellt werden, die das Vorliegen/Fehlen einer Krankheit und/oder des Entwicklungsgrads der Krankheit als Eingabedaten bereitgestellt werden, die durch Unterabschnitte bestimmt werden, die Größen, Geometrien und/oder die Morphologie von Muskel-Skelett-Strukturen von orthopädischem Interesse und insbesondere von Strukturen des anatomischen Wirbelsäulenbereichs und gegebenenfalls weitere Daten bestimmen, die durch verschiedene Untersuchungen und/oder Personendaten oder die Krankengeschichte des Patienten erhalten werden.

12. Einrichtung nach einem oder mehreren der vorhergehenden Ansprüche, **dadurch gekennzeichnet, dass** sie mit Mitteln zum Speichern von bekannten klinischen Fällen und/oder diagnostischen Bildern und/oder Verarbeitungsdaten und/oder diagnostischen Daten von vorausgehenden Untersuchungen betreffend eine orthopädische Krankheit und gegebenenfalls auch andere Krankheiten für jeden Patienten versehen ist, die zu verschiedenen Zeitpunkten auch innerhalb von relativ langen Zeitintervallen, wie Tagen, Monaten oder Jahren, erfasst wurden, wobei die Daten zur Bestimmung mindestens eines oder mehrerer Referenzwerte zur Unterscheidung zwischen dem Vorliegen/Fehlen einer Krankheit und/oder einer Skala von numerischen Werten für verschiedene Entwicklungsgrade der Krankheit verwendet werden, und wobei die Werte mit Größen-, geometrischen und morphologischen Werten von Strukturen der Wirbelsäule zur Bestimmung der Entwicklung, d. h. der Nachsorge, einer Krankheit und der Art von auszuführendem Eingriff und/oder zur Überprüfung der Wirksamkeit einer gerade vorgenommenen Therapie verglichen werden.

13. Einrichtung nach Anspruch 1, **dadurch gekennzeichnet, dass** die Einheit zur Erkennung von Bildern durch kernmagnetische Resonanz aus einer Magnetstruktur besteht, die einen Hohlraum mit einer Öffnung für das Einführen des untersuchten Körperteils oder durch die der Patient einfach selbst durch Hineinlaufen eintreten kann oder durch die er auf beweglichen Trägermitteln, wie einem Untersuchungstisch, einem Rollstuhl oder einer ähnlichen Vorrichtung, transportiert werden kann, aufweist, so dass der zu untersuchende Körperteil, insbesondere Teile des Muskel-Skelett-Apparats, wie die Wirbelsäule für den lumbosakralen und zervikalen Teil, in dem Detektionshohlraum platziert wird, um die Erfassung von Bildern des anatomischen Bereichs von Interesse in einem Ruhezustand oder unter Last zu gestatten.

14. Einrichtung nach Anspruch 12, **dadurch gekennzeichnet, dass** sie Mittel zum Positionieren und/oder Anlehnen und/oder Halten und/oder Stützen des Körpers des Patienten oder des untersuchten Körperteils aufweist, die so vorgesehen sind, dass sie innerhalb des Hohlraums, in dem der Patient oder der untersuchte Körperteil aufgenommen ist, aufgebaut und abgebaut und/oder bewegt werden können, um den Patienten bequem zu positionieren und um den Signal-Rausch-Abstand präzise zu optimieren.

15. Einrichtung nach Anspruch 13, 14, **dadurch gekennzeichnet, dass** der Magnet und die Mittel zum Positionieren und/oder Anlehnen und/oder Halten und/oder Stützen des Körpers des Patienten oder des untersuchten Körperteils sich zusammen drehen, so dass der Patient bequem in dem Detektionshohlraum aufgenommen werden kann, zum Beispiel auf einem Untersuchungstisch zuerst in Rückenlage und dann, nach der Drehung, in der aufrechten Position und/oder in Zwischenpositionen liegen kann, wobei orthopädische Strukturen, wie die Wirbelsäule, der natürlichen Last unterworfen sind.

16. Einrichtung nach den Ansprüchen 13 bis 15, **dadurch gekennzeichnet, dass** die Mittel zum Positionieren und/oder Anlehnen und/oder Halten und/oder Stützen des Körpers des Patienten oder des untersuchten Körperteils Aufnahme- und/oder Durchlaufführungen für verschiedene Vorrichtungen, wie Mittel zum Empfangen von Resonanzsignalen, aufweisen.

17. Einrichtung nach den Ansprüchen 13 bis 16, **dadurch gekennzeichnet, dass** die Mittel (23) zum Empfangen von Resonanzsignalen beweglich direkt an dem untersuchten Körper oder an einem Teil von diesem befestigt werden können und/oder in Wänden (202) integriert sind, die Pfosten (201) bilden oder tragen.

18. Einrichtung nach den Ansprüchen 13 bis 17, **dadurch gekennzeichnet, dass** die Mittel zum Positionieren und/oder Anlehnen und/oder Halten und/oder Stützen des Körpers des Patienten oder des untersuchten Körperteils aus einem Untersuchungstisch (22) oder dergleichen bestehen, der mit Mitteln zur Einstellung der Höhe der Tragebene (222) für den Patienten und/oder der Neigung um eine oder mehrere Achsen versehen ist, so dass der Patient gemäß verschiedenen Ausrichtungen im Raum bewegt wird, wobei die Mittel insbesondere gestatten, die Neigung der Tragebene (222) einzustellen, so dass der Untersuchungstisch (22) von einer horizontalen Position zu einer im Wesentlichen aufrechten Position gebracht wird, wobei der Untersuchungstisch (22) gemäß unterschiedlichen Neigungsgraden angehalten wird, um MRT-Bilder des anatomischen Bereichs der Wirbelsäule oder von anderen orthopädischen Bereichen gemäß verschiedenen Belastungsgraden zu erfassen, bis der anatomische Bereich von Interesse komplett belastet wird, wenn der Tisch in eine vollständig vertikale Position gebracht wird.

19. Einrichtung nach den Ansprüchen 13 bis 18, **dadurch gekennzeichnet, dass** die Mittel zum Positionieren und/oder Anlehnen und/oder Halten und/oder Stützen des Körpers des Patienten oder des untersuchten Körperteils aus einem Stuhlelement (26) bestehen, das auf bewegliche Weise und in verschiedenen Positionen innerhalb des Untersuchungshohlraums montiert oder platziert werden kann, und es besitzt Mittel zur Einstellung der Höhe des Sitzes, der entsprechend mindestens einer Achse geneigt werden kann, die in Richtung des Magnetfelds ausgerichtet ist, und mindestens ein Rückenteil, das entsprechend mindestens einer Achse geneigt werden kann, die in Richtung des Magnetfelds ausgerichtet ist.

20. Einrichtung nach den Ansprüchen 13 bis 19, **dadurch gekennzeichnet, dass** die Mittel zum Positionieren und/oder Anlehnen und/oder Halten und/oder Stützen des Körpers des Patienten oder des untersuchten Körperteils aus einer oder mehreren Fußstützen mit unterschiedlichen Höhen und/oder mit einstellbaren Höhen besteht, die beweglich und in verschiedenen Positionen an der Basis (202) oder auf der Auftrittebene der Magnetstruktur innerhalb des Detektionshohlraums montiert werden können.

21. Einrichtung nach den Ansprüchen 13 bis 20, **dadurch gekennzeichnet, dass** die Mittel zum Positionieren und/oder Anlehnen und/oder Halten und/oder Stützen des Körpers des Patienten oder des untersuchten Körperteils individuell oder in Kombination mit anderen Positionierungsmitteln vorgesehen sind, bestehend aus einem oder mehreren Haltegriffen (25) oder aus anderen Haltemitteln, die auf feste oder bewegliche Weise und in verschiedenen Positionen innerhalb des Detektionshohlraums befestigbar vorgesehen sind.

## Revendications

1. Appareil pour déterminer des indications d'assistance à la diagnose de pathologies orthopédiques, comprenant une section de détection et d'acquisition de signaux d'un corps examiné ou d'une partie de celui-ci, comprenant par ailleurs une section de détection et une section d'acquisition d'images par résonance magnétique nucléaire (RMN), dans lequel la section de détection est une unité de détection d'images par résonance magnétique nucléaire et la section d'acquisition d'images par RMN est une unité d'acquisition d'images par RMN ayant des moyens de balayage RM dont la forme et les dimensions permettent l'acquisition d'images RM de la région anatomique d'intérêt orthopédique et notamment de la région de la colonne vertébrale, notamment de la colonne vertébrale dans la zone lombo-sacrée et/ou la zone cervicale, et ladite unité de détection d'images par RMN est intégrée dans une section de traitement d'images acquises dans la section de traitement par rapport à des données d'image et/ou à des signaux de résonance, de manière que ladite unité d'acquisition d'images par résonance magnétique nucléaire et ladite section de traitement d'images acquises sont groupées dans un seul appareil, laquelle section de traitement définit, à partir de données d'image et/ou de signaux de résonance, des valeurs d'un ou plusieurs paramètres numériques différents comme des paramètres géométriques, concernant les dimensions et/ou la forme et/ou le volume et/ou les géométries des structures muscolo-squelettiques de la colonne vertébrale, indiquant la présence ou l'absence d'une pathologie orthopédique et/ou une mesure de la condition d'évolution de ladite pathologie orthopédique, dont les fonctionnalités de traitement sont réalisées en mode « en ligne » par le balayage RM, c'est-à-dire immédiatement après l'acquisition des données d'image et les fonctionnalités d'acquisition de ladite section d'acquisition d'images sont guidées par la section de traitement elle-même au moyen d'une ligne de rétroaction, dans lequel la section de traitement comprend une sous-section avec des moyens pour déterminer les paramètres géométriques des structures muscolo-squelettiques principales d'intérêt orthopédique de la colonne vertébrales, notamment des vertèbres, des structures des disques et/ou du canal spinal, lesdits paramètres géométriques étant détectés pour des degrés d'inclinaison différents de la colonne vertébrale, notamment avec la colonne vertébrale dans une position sensiblement horizontale, c'est-à-dire en condition de repos, et/ou dans une position sensiblement verticale, c'est-à-dire sous charge et/ou en positions intermédiaires entre les positions horizontale et verticale de la colonne vertébrale.

2. Appareil selon la revendication 1, **caractérisé en ce que** la section de traitement comprend une sous-section avec des moyens pour déterminer les paramètres géométriques concernant la position dans l'espaces des structures principales de la colonne vertébrale, notamment des vertèbres, des structures des disques et/ou du canal spinal l'un par rapport à l'autre dans une position sensiblement horizontale, c'est-à-dire en condition de repos, et/ou dans une position sensiblement verticale, c'est-à-dire sous charge et/ou en positions intermédiaires entre les positions horizontale et verticale de la colonne vertébrale.

3. Appareil selon l'une ou plusieurs des revendications précédentes, **caractérisé en ce que** la section de traitement comprend une sous-section avec des moyens pour comparer lesdits paramètres avec une valeur de référence pour discriminer la présence/l'absence d'une condition pathologique et/ou pour la comparaison avec une échelle de référence pour déterminer un paramètre indiquant le degré d'évolution de la condition pathologique, laquelle valeur de référence pour discriminer la présence/l'absence de la condition pathologique et/ou laquelle échelle de référence pour déterminer le degré d'évolution de la condition pathologiques sont comprises dans une base de données de cas cliniques connus.

4. Appareil selon l'une ou plusieurs des revendications précédentes, **caractérisé en ce que** la section de traitement comprend une sous-section avec des moyens pour déterminer les valeurs numériques de ladite condition pathologique des structures muscolo-squelettiques d'intérêt orthopédique, comme des articulations ou d'autres et notamment des structures de la colonne vertébrale, concernant la présence/l'absence de celle-ci et la condition d'évolution de la pathologie au moyen de l'analyse comparative du contraste de l'image RM comme la détection de cartes de T1 ou de T2 et/ou l'analyse d'images au moyens d'agents de contraste appropriés, obtenus à des étages différents de la condition pathologique à partir d'images propres à des patients faisant partie de la base de données de cas cliniques connus et/ou d'images propres à des examens précédents effectués sur le même patient.

5. Appareil selon l'une ou plusieurs des revendications précédentes, **caractérisé en ce que** la section de traitement comprend une sous-section avec des moyens pour déterminer les valeurs numériques de ladite condition pathologique des structures de la colonne vertébrale concernant la présence/l'absence de celle-ci et la condition d'évolution de la pathologie par le traitement des données d'image au moyens d'algorithmes de classification et/ou de prédiction, instruit par des procédés d'instruction et d'essai, au moyen de données d'image concernant la colonne vertébrale de cas cliniques connus compris dans une base de données de cas cliniques connus.

6. Appareil selon l'une ou plusieurs des revendications précédentes, **caractérisé en ce que** la section de traitement comprend une sous-section pour déterminer l'état pathologique des structures muscolo-squelettiques d'intérêt orthopédique et des structures de la colonne vertébrale, par des moyens de segmentation d'images comme des moyens de rendu éventuellement combinés avec des moyens de morphing et/ou de lissage, pour déterminer des sous-ensembles de données d'image et/ou des pixels et/ou des voxels et pour identifier des objets réels représentés dans l'image par lesdits sous-ensembles de données d'image et/ou de pixels et/ou de voxels, le sous-ensemble de pixels ou de voxels ou de données d'image représentant les structures principales relatives à la région anatomique de la colonne vertébrale, notamment des vertèbres et des structures des disques et les dimensions et/ou la forme et/ou les géométries desdites structures déterminées étant identifié.

7. Appareil selon la revendication 6, **caractérisé en ce que** la sous-section pour déterminer les dimensions, la géométrie et/ou la morphologie des structures orthopédiques de la région anatomique examinée, dans le cas de la région anatomique de la colonne vertébrale, notamment des vertèbres et des disques intervertébraux, comprend des moyens pour comparer les données dimensionnelles, géométriques et morphologiques des structures principales de la région examinée avec les données dimensionnelles, géométriques et morphologiques d'une pluralité de cas cliniques connus, la comparaison offrant des informations sur les différences et les identités des dimensions et/ou de la géométrie et/ou de la morphologie des structures principales de la région examinée par rapport aux dimensions et/ou aux géométries et/ou à la morphologie desdites structures d'un ou plusieurs cas cliniques connus, et offre donc une indication sur la présence/l'absence d'une pathologie orthopédique et/ou une indication sur la condition d'évolution de ladite pathologie orthopédique, par exemple pour mettre en évidence des restrictions éventuelles du canal spinal et/ou l'instabilité des vertèbres, dans le cas de la région anatomique de la colonne vertébrale.

8. Appareil selon la revendication 6, **caractérisé en ce qu'**il comprend des moyens pour vérifier la fiabilité de l'identification du sous-ensemble de pixels ou de voxels ou de données d'image représentant dans l'image ou dans l'ensemble de données d'image un objet réel et, dans le cas de la région anatomique de la colonne vertébrale, notamment des vertèbres, des structures des disques et/ou du canal spinal, ces moyens de vérification comprenant une base de données de configuration dimensionnelles, géométriques et/ou volumétriques et/ou morphologiques, c'est-à-dire des formes typiques des structures principales de la région examinée en conditions spécifiques d'absence ou de présence d'une pathologie et/ou d'un degré spécifique d'évolution de la pathologie, tandis que les dimensions, la géométrie et/ou le volume et/ou la morphologie de structures examinées, déterminés par des sous-sections de moyens de traitement d'images d'un patient examiné sont comparés avec lesdites données dimensionnelles, géométriques et/ou volumétriques typiques et/ou avec les morphologies typiques desdites structures, qui sont en forme de valeur moyennes et/ou d'intervalles de valeur moyennes de différences dimensionnelles, géométriques et/ou volumétriques et/ou morphologiques, un premier seuil maximal de différence étant déterminé, le dépassement duquel comporte que les données dimensionnelles, géométriques et/ou volumétriques et/ou morphologiques soient considérés incompatibles et peu fiables, les moyens de vérification étant pourvus d'une sous-section pour signaler ou demander la répétition du procédé pour déterminer les caractéristiques dimensionnelles et/ou géométriques et/ou volumétriques et/ou morphologiques et/ou d'acquisition de l'image ou des images RM, ou lesdits moyens de vérification sont pourvus d'une sous-section pour commander automatiquement la répétition du procédé pour déterminer les caractéristiques dimensionnelles et/ou géométriques et/ou volumétriques et/ou morphologiques et/ou l'acquisition de l'image ou des images RM.

9. Appareil selon l'une ou plusieurs des revendications précédentes, **caractérisé en ce que** la section de détection, notamment l'unité de détection d'images par résonance magnétique nucléaire est connectée à la section de traitement des images acquises par une ligne de rétroaction.

10. Appareil selon l'une ou plusieurs des revendications précédentes, **caractérisé en ce que** la section de traitement comprend des moyens pour analyser les images acquises par rapport à des paramètres de qualité prédéterminés desdites données d'image et/ou de signaux de résonance et des moyens pour changer automatiquement les réglages d'acquisition et/ou les paramètres d'acquisition des données d'image, qui sont commandés par lesdits moyens pour analyser les données d'image sur la base des paramètres de qualité des données d'image et/ou des signaux de résonance à partir desquels ils sont déterminés.

11. Appareil selon l'une ou plusieurs des revendications précédentes, **caractérisé en ce que** la section de traitement comprend une sous- section pour déterminer l'état pathologique et/ou la présence de dommages aux structures orthopédiques, comprenant des moyens de classification et/ou de prédiction comme, par exemple, un algorithme de classification et/ou de prédiction instruit avec les données d'une base de données de cas cliniques connus, recevant, comme données d'entrée, des paramètres indiquant la présence/l'absence d'une pathologie et/ou le degré d'évolution de la pathologie déterminés par les sous-sections pour déterminer les dimensions, les géométries et/ou la morphologie des structures muscolo-squelettiques d'intérêt orthopédique et notamment les structures de la région anatomique de la colonne vertébrale et éventuellement d'autres données obtenues par des examens différents et/ou des données personnelles de l'histoire médicale du patient.

12. Appareil selon l'une ou plusieurs des revendications précédentes, **caractérisé en ce qu'**il est pourvu de moyens pour stocker des cas cliniques et/ou des images diagnostiques et/ou des données de traitement et/ou des données diagnostiques connus d'examens précédents propres à une pathologie orthopédique et éventuellement aussi à d'autres pathologies pour chaque patient, acquis en différents moments, même sur des intervalles de temps relativement longs comme des jours, des mois, ou des années, lesdites données étant utilisées pour déterminer au moins une ou plusieurs valeurs de référence pour discriminer la présence/l'absence d'une pathologie et/ou une échelle de valeurs numériques pour de différents degrés d'évolution de la pathologie, et lesdites valeurs étant comparées avec les valeurs dimensionnelles, géométriques et morphologiques des structures de la colonne vertébrale pour déterminer l'évolution, c'est-à-dire le suivi d'une pathologie et le type d'intervention à effectuer et/ou pour vérifier l'efficacité d'une thérapie en cours.

13. Appareil selon la revendication 1, **caractérisé en ce que** l'unité de détection d'images par résonance magnétique nucléaire est composée d'une structure magnétique qui définit une cavité ayant une ouverture pour l'introduction de la partie du corps examiné ou à travers laquelle le patient peut entrer par déambulation autonome ou transporté sur un moyen de support mobile comme une table d'examen, un fauteuil roulant ou d'autres dispositifs similaires, de façon que la cavité de détection contienne la partie du corps à examiner, notamment les parties du système muscolo-squelettique, comme la colonne vertébrale dans les zones lombo-sacrée et cervicale, pour permettre l'acquisition d'images de la région anatomique d'intérêt à repos ou sous charge.

14. Appareil selon la revendication 12, **caractérisé en ce qu'**il comprend des moyens de positionnement et/ou d'appui et/ou de retenue et/ou de support du corps du patient ou de la partie du corps examinée, ces moyens étant aptes à être assemblés ou désassemblés et/ou déplacés à l'intérieur de la cavité de logement du patient ou de la partie du corps examinée pour un positionnement confortable du patient et une optimisation précise du rapport signal-bruit.

15. Appareil selon la revendication 13, 14, **caractérisée en ce que** l'aimant et les moyens de positionnement et/ou d'appui et/ou de retenue et/ou de support du corps du patient ou de la partie du corps examinée tournent ensemble de façon que la patient puisse être logé confortablement à l'intérieur de la cavité de détection, par exemple couché sur la table d'examen d'abord sur le dos, et en suite, après une rotation, en position verticale et/ou en position intermédiaires, dans lequel les structures orthopédiques comme la colonne vertébrales sont sous charge naturelle.

16. Appareil selon les revendication 13 à 15, **caractérisé en ce que** les moyens de positionnement et/ou d'appui et/ou de retenue et/ou de support du corps du patient ou de la partie du corps examinée comprennent des guides de logement et/ou de passage pour des dispositifs différents, comme des moyens de réception de signaux de résonance.

17. Appareil selon les revendications 13 à 16, **caractérisé en ce que** les moyens (23) de réception de signaux de résonance peuvent être fixées de façon mobile sur le corps examiné ou sur une partie de celui-ci est/ou sont intégrés dans des parois (202) constituant ou portant des pôles (201).

18. Appareil selon les revendications 13 à 17, **caractérisé en ce que** les moyens de positionnement et/ou d'appui et/ou de retenue et/ou de support du corps du patient ou de la partie du corps examinée sont constitués d'une table d'examen (22) ou similaire, pourvue de moyens pour régler la hauteur du plan de support (222) du patient et/ou pour l'incliner autour d'un ou plusieurs axes de manière que le patient soit déplacé suivant plusieurs orientations différentes dans l'espace, et notamment lesdits moyens permettent de régler l'inclinaison du plan de support (222) de manière que la table d'examen (22) passe d'une position horizontale à une position sensiblement verticale, d'arrêter la table d'examen (22) sur des degrés d'inclinaison différents, de manière à acquérir des images RM de la région anatomique de la colonne vertébrale ou d'autres régions orthopédiques sur des degrés de charge différents, jusqu'à la charge complète de la région anatomique d'intérêt quand la table d'examen est amenée en position complètement verticale.

19. Appareil selon les revendications 13 à 18, **caractérisé en ce que** les moyens de positionnement et/ou d'appui et/ou de retenue et/ou de support du corps du patient ou de la partie du corps examinée sont constitués d'un élément de fauteuil (26), qui peut être monté ou placé de façon mobile et en plusieurs positions différentes à l'intérieur de la cavité d'examen, et comporte des moyens de réglage de la hauteur du siège pouvant peut être inclinés suivant au moins un axe orienté dans la direction du champ magnétique est au moins une partie arrière pouvant être inclinés suivant au moins un axe orienté dans la direction du champ magnétique.

20. Appareil selon les revendications 13 à 19, **caractérisé en ce que** les moyens de positionnement et/ou d'appui et/ou de retenue et/ou de support du corps du patient ou de la partie du corps examinée sont constitués d'un ou plusieurs repose-pieds ayant des hauteurs différentes ou des hauteurs réglables, et pouvant être montés mobiles en plusieurs positions différentes sur la base (202) ou sur le plan de piétinement de la structure magnétique à l'intérieur de la cavité de détection.

21. Appareil selon les revendications 13 à 20, **caractérisé en ce que** lesdits moyens de positionnement et/ou d'appui et/ou de retenue et/ou de support du corps du patient ou de la partie du corps examinée sont prévus seuls ou en association avec d'autres moyens de positionnement constitués d'une ou plusieurs poignées de retenue (25) ou d'autres moyens de retenue prévus fixes ou mobiles et pouvant être fixés en plusieurs positions différentes à l'intérieur de la cavité de détection (21).
